(19) Europäisches Patentamt
European Patent Office
Office européen des brevets

(11) Veröffentlichungsnummer: **0 241 428 B1**

(12) **EUROPÄISCHE PATENTSCHRIFT**

(45) Veröffentlichungstag der Patentschrift: **18.03.92**

(51) Int. Cl.$^5$: **A61K 31/44**

(21) Anmeldenummer: **87810213.6**

(22) Anmeldetag: **06.04.87**

(54) **Verwendung von 2-Aminoalkyl-5-pyridinol-Verbindungen als Nootropika und Antidepressiva.**

(30) Priorität: **11.04.86 CH 1432/86**

(43) Veröffentlichungstag der Anmeldung:
**14.10.87 Patentblatt 87/42**

(45) Bekanntmachung des Hinweises auf die Patenterteilung:
**18.03.92 Patentblatt 92/12**

(84) Benannte Vertragsstaaten:
**BE CH DE FR GB IT LI LU NL SE**

(56) Entgegenhaltungen:
**EP-A- 0 019 739**

**DRUG DEVELOPMENT RESEARCH, Band 5, Nr. 4, 1985, Seiten 327-336, New York, US; S.W. SCHAFFER et al.: "Identification of a new cardioprotective agent-6(2-isopropylaminopropyl)-3-pyridinol".**

**FED. PROC., Band 43, Nr. 3, 1984, Seite 356, Zusammenfassungs Nr. 417, Washington, US; B.H. TAN et al.: "Identification of a new cardioprotective agent: CGS 5649B 6(2-isopropyl amino-propyl)-3-pyridinol".**

**FED. PROC., Band 43, Nr.3, 1984, Zusammenfassungs Nr. 1487, Washington, US; H. J. POVALSKI et al.: "Moderation of myocardial ischemic injury by CGS 5649B".**

**FED. PROC., Band 45, Nr. 3, März 1, 1986, Seite 197, Zusammenfassungs Nr. 248, Washington, US; H.H. OEI et al.: "Further characterization of the cardioprotective effects of CGS 5649B (6(2-isopropylaminopropyl)-3-pyridinol)".**

(73) Patentinhaber: **CIBA-GEIGY AG**
**Klybeckstrasse 141**
**CH-4002 Basel(CH)**

(72) Erfinder: **Mondadori, Cesare, Dr.**
**Traugott-Meyer-Strasse 70**
**CH-4147 Aesch(CH)**

EP 0 241 428 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von sekundären 2-Aminoalkyl-5-pyridinolen der allgemeinen Formel I

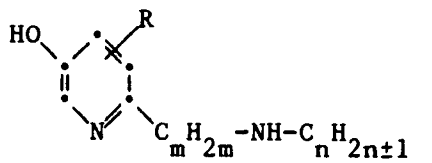

(I),

worin R Wasserstoff oder Niederalkyl bedeutet, m für eine ganze Zahl von 2 bis 4 steht, und n eine ganze Zahl von 1 bis 7 bedeutet, oder ihren therapeutisch verwendbaren Säureadditionssalzen zur Herstellung von nootropisch und antidepressiv wirksamen Arzneimitteln zur therapeutischen und prophylaktischen Behandlung von celebraler Insuffizienz.

Der Ausdruck "nieder" definiert in den oben oder nachfolgend genannten organischen Resten oder Verbindungen solche mit höchstens 7, vorzugsweise 4, insbesondere 1 oder 2 Kohlenstoffatomen.

Bedeutet R Niederalkyl, so kann diese Gruppe in irgendeiner der freien Stellungen 2-, 4- oder 6- des Pyridinrestes stehen. Der Niederalkylrest ist z.B. Aethyl, n- oder Isopropyl, n- oder Isobutyl, n-Pentyl, n-Hexyl oder n-Heptyl, vorzugsweise jedoch Methyl. Das Symbol R bedeutet jedoch in erster Linie Wasserstoff.

Die Alkylengruppe $C_mH_{2m}$ bedeutet vorzugsweise 1,2-Propylen, aber auch Aethylen, 1,3-Propylen, 1,2-, 1,3- oder 1,4-Butylen.

Die Gruppe $C_nH_{2n+1}$ steht vorzugsweise für Niederalkyl, z.B. Methyl, Aethyl, n- oder iso-Propyl, n-, iso- oder tert.-(Butyl, Pentyl, Hexyl oder Heptyl); insbesondere für Isopropyl.

Die genannte Gruppe $C_nH_{2n-1}$ bedeutet entweder Niederalkenyl, z.B. Allyl, Methallyl, 2- oder 3-(Butenyl, Pentenyl, Hexenyl oder Heptenyl), oder auch ringgeschlossenes niederes Cycloalkyl, z.B. Cyclopropyl, Cyclobutyl, Cyclopentyl, Cyclohexyl oder Cycloheptyl; insbesondere jedoch Cyclopropyl oder Cyclohexyl.

Die oben genannten Verbindungen der Formel I sind bekannt und beispielsweise im EP-Patent Nr. 19739 als antihypertensiv, aber insbesondere als kardioprotektiv, wie z.B. antiischämisch (d.h. antianginal) wirksam beschrieben worden.

Die Säureadditionssalze der dibasischen Verbindungen der Formel I sind insbesondere therapeutisch verwendbare Säureadditionssalze. Säuren, die therapeutisch verwendbare Säureadditionssalze ergeben, sind z.B. anorganische Säuren, wie Halogenwasserstoffsäuren, z.B. Chlorwasserstoff- oder Bromwasserstoffsäure, oder Schwefel-, Phosphor-, Salpeter- oder Perchlorsäure; oder organische Säuren, wie aliphatische oder aromatische Carbon- oder Sulfonsäuren, z.B. Ameisen-, Essig-, Propion-, Bernstein-, Glykol-, Milch-, Fumar-, Aepfel-, Wein-, Zitronen-, Malein-, Hydroxymalein-, Brenztrauben-, Phenylessig-, Benzoe-, 4-Aminobenzoe-, Anthranil-, 4-Hydroxybenzoe-, Salicyl-, Pamoe-, Nikotin-, Methansulfon-, Aethansulfon-, Hydroxyäthansulfon-, Aethylensulfon-, Benzolsulfon-, Halogenbenzolsulfon-, Toluolsulfon-, Naphthalinsulfon-, Sulfanil-, Cyclohexylsulfaminsäure; oder Ascorbinsäure; die Salze sind bekannt und beispielsweise im EP-Patent Nr. 19739 beschrieben worden.

Es wurde nun überraschenderweise gefunden, dass die oben genannten Verbindungen der Formel I nootropisch und antidepressiv wirksam sind und beispielsweise einen Schutz vor amnesiogener Wirkung eines cerebralen Elektroschocks und eine Verbesserung der Gedächtnisleistung sowie Konzentrationsfähigkeit bewirken. Die vigilanzstimulierenden Wirkungen, Verbesserungen der Konzentrationsfähigkeit und antidepressive Effekte sind gekoppelt mit einer sehr guten Verträglichkeit und sehr niedriger Toxizität.

Aufgrund dieser Eigenschaften sind die Verbindungen der Formel I zur Behandlung von cerebraler Leistungsinsuffizienz, insbesondere von Gedächtnisstörungen verschiedener Genese, Insuffizienz der Vigilanzregulation, wie senile Demenz, Multiinfarkt-Demenz oder Demenz vom Alzheimer-Typ, ferner von Folgeerscheinungen von Hirntraumen oder Apoplexie sowie Altersdepressionen geeignet. Die Verbindungen werden enteral oder parenteral, vorzugsweise oral oder intravenös, z.B. durch Gelatine-Kapseln oder in Form von Stärke enthaltenden Suspensionen bzw. wässrigen Lösungen, verabreicht. Die verwendete Dosis kann in einem Bereich zwischen 1 bis 30 mg/kg/Tag i.p., oder zwischen 10 und 100 mg/kg/Tag bei peroraler Verabreichung liegen.

Die erfindungsgemässen Verbindungen der Formel I zeigen auch deutliche Wirkung auf den Vigilanzzustand von Katzen. Die Gesamtschlafzeit wurde dosisabhängig verkürzt. Der relative Anteil an REM-Schlaf (Rapid-Eye-Movement-Sleep), der verschiedentlich mit Gedächtnisaktivitäten in Verbindung gebracht wird,

wurde interessanterweise nicht verkleinert im Gegenteil im Vergleich zur Gesamtschlafzeit stark verlängert. Dies ist umso bemerkenswerter, als dass praktisch alle psychoaktiven Substanzen REM-Schlaf-verkürzend wirken.

Als Beispiel für Illustration der Erfindung sei das 2-(2-Isopropylaminopropyl)-5-pyridinol-monofumarat genannt, welches die Gesamtschlafzeit bei einer Dosis von 10 mg/kg p.o. um 28 % und bei einer Dosis von 30 mg/kg p.o. um 54 % verkürzt.

Die vorliegende Erfindung betrifft insbesondere die Verwendung von Verbindungen der Formel I, worin R Wasserstoff oder Methyl bedeutet, m für die ganze Zahl 2 oder 3 steht, und n eine ganze Zahl von 2 bis 6 bedeutet, und ihren Säureadditionssalzen, insbesondere therapeutisch verwendbaren Säureadditionssalzen zur Herstellung eines nootropisch und stimulierend antidepressiv wirksamen Arzneimittels.

Besonders hervorzuheben ist die Verwendung von Verbindungen der Formel II

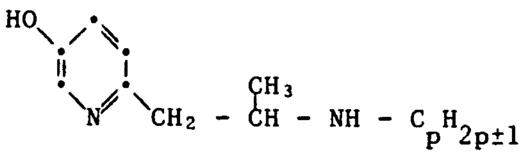

(II),

worin p eine ganze Zahl von 3 bis 6 bedeutet und ihren Säureadditionssalzen, insbesondere therapeutisch verwendbaren Säureadditionssalzen zur Herstellung eines nootropisch wirksamen Arzneimittels.

Bevorzugt ist die Verwendung von Verbindungen der allgemeinen Formel II, worin $C_pH_{2p\pm1}$, tert.-Butyl, Allyl oder Cyclopropyl oder in erster Linie Isopropyl bedeutet, und ihren Säureadditionssalzen, insbesondere pharmazeutisch verwendbaren Säureadditionssalzen zur Herstellung eines nootropisch wirksamen Arzneimittels.

Die Herstellung der Verbindungen der Formel I und/oder II wird z.B. im EP-Patent Nr. 19739 ausführlich beschrieben.

Die Verbindungen der Formel I und II können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzusweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcelullose und/oder Polyvinylpyrrolidon, und, wenn erwünscht, Sprengmitel, z.B. Stärken, Agar, Alginsäure oder ein Salz davon, Enzyme der Bindemittel und/oder Brausemischungen, oder Adsorptionsmittel, Farbstoffe, Geschmackstoffe und Süssmittel, Injizierbare Präparate sind vorzugsweise isotonische wässerige Lösungen oder Suspensionen, und Suppositorien in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weiter pharmakologisch wertvolle Stoffe enthalten können, werden in an sich bekannter Weise, z.B. mittels konventioneller Misch-, Granulier- oder Dragierverfahren, hergestellt und enthalten von 1 % bis 75 %, insbesondere von 10 % bis 50 %, des Aktivstoffes.

Die folgenden Beispiele dienen zur Illustration der Erfindung.

Beispiel 1: Reduktion der amnesiogenen Wirkung eines cerebralen Elektroschocks [Methode nach Mondadori C. und Classen W., Acta Neurol. Scand. 69, Suppl. 99, 125-129 (1984)].

Die Testvorrichtung besteht aus einer grossen Box (35 x 20 x 10 cm), die durch eine Schiebetür mit einer kleinen Box (10 x 10 x 18 cm) verbunden ist. Während die kleine Box durch eine 100 Watt Lampe von oben hell erleuchtet wird, ist die grosse Box dunkel. Der Boden beider Abteile besteht aus einem elektrisch leitenden Gitterrost, dessen Gitterstäbe (6 mm Durchmesser) in einem Abstand von jeweils 13 mm angeordnet sind. Zum Training werden Gruppen von jeweils 10 männlichen Mäusen mit einem Körpergewicht von 20-22 g einzeln in die hell erleuchtete Box gesetzt. Da Mäuse eine Spontanpräferenz für Dunkel haben, gehen die meisten innerhalb von 30 Sekunden ins dunkle Abteil. Sobald sie dieses vollständig betreten haben, wird die Schiebetür geschlossen und ein Fussschock (1 mA, 50 Hz, 5 Sekunden)

verabreicht. Die Tiere werden darauf sofort aus dem Versuchsapparat entfernt. Zwei separate Versuche werden durchgeführt (vormittags zwischen 8-11 und nachmittags zwischen 12-15 Uhr).

Zur Prüfung des Lerneffektes (Retest) werden die Mäuse nach 24 Stunden wiederum einzeln ins helle Abteil gesetzt und die Zeitspanne, bis sie sich ganz im dunklen Abteil befinden, gemessen. Ueblicherweise bleiben nun die meisten Tiere über die ganze Beobachtungszeit von 150 Sekunden im hellen Abteil. Der Lerneffekt wird weitgehend aufgehoben bzw. die Erinnerung an den Fussschock mindestens partiell ausgelöscht, wenn als amnesiogene Behandlung unmittelbar anschliessend an den Fussschock des Lerndurchgangs eine temporale Elektroschockbehandlung angeschlossen wird. Parameter des Elektroschocks: 50 mA, 0,4 Sekunden, 50 Hz.

Zur Prüfung und zum Vergleich ihrer Schutzwirkung gegenüber der amnesiogenen Wirkung des Elektroschocks werden den in verschiedene Gruppen aufgeteilten Tieren die Testsubstanzen 30 Minuten vor dem Lerndurchgang in Dosen von 0,1 mg/kg, 1 mg/kg, 10 mg/kg und 30 mg/kg (für jede Dosis werden 10 Mäuse verwendet) verabreicht. Als Lösung wird 0,5 % Methylcellulose (Methocel) oder die Lösung alleine (= Placebo) intraperitoneal verabreicht und die Tiere unmittelbar nach dem Lerndurchgang der Elektroschockbehandlung unterzogen. 24 Stunden später wird anhand der Verweildauer in der beleuchteten Box das Ausmass des noch erhalten gebliebenen Lerneffektes gemessen und mit demjenigen der anderen Testsubstanzen wie auch von Kontrolltieren mit alleiniger Verabreichung des jeweils verwendeten Lösungsmittels und Training ohne, wie auch solchen mit anschliessender Elektroschockbehandlung, verglichen. Eine mit dem Placebo behandelte Gruppe ohne Elektroschockbehandlung dient zur Kontrolle des Lernens im Vermeidungstest, eine zweite mit Placebo behandelte Kontrollgruppe mit anschliessender Elektroschockbehandlung dient zur Ueberwachung des Ausmasses der amnesiogenen Wirkung des Elektroschocks.

Die oben erwähnten Wirkungen lassen sich beispielsweise nach intraperitonealer Verabreichung von 0,1 mg/kg 2-(2-Isopropylaminopropyl)-5-pyridinol-monofumarat nachweisen.

Beispiel 2: Messung der Verbesserung der Gedächtnisleistung [Methode nach Mondadori C., step down passive avoidance test, Psychopharmacol. 63, 297-300 (1979)].

Die Testvorrichtung besteht aus einer mit Tageslicht normal beleuchteten Box (50 x 50 x 50 cm) mit einem elektrisch leitenden Gitterrost, dessen Gitterstäbe (4 mm Durchmesser) in einem Abstand von jeweils 13 mm angeordnet sind. In der Mitte des Gitterrostes ist eine hölzerne Plattform (12 mm Höhe, 67 mm innerer Durchmesser) angebracht, welche von einer Plastikröhre (18 cm hoch, 68 mm Durchmesser) umgeben ist. Zur Durchführung des Trainings werden Gruppen von jeweils 50 männlichen Mäusen mit einem Körpergewicht von 20-22 g verwendet, indem jeweils ein Tier auf die von der Plastikröhre umgebene Plattform gesetzt, nach 10 Sekunden die Plastikröhre entfernt und die Zeitdauer gemessen wird, welche das Tier zum Hinabsteigen und Berühren des Gitterrostes mit allen 4 Pfoten benötigt. Wenn alle 4 Pfoten den Gitterrost berühren, wird ein Fussschock (1 mA, 50 Hz, 1 Sekunde) verabreicht.

Zur Prüfung des Lerneffektes (Retest) werden die Mäuse nach 24 Stunden wiederum einzeln auf die von der Plastikröhre umgebene Plattform gesetzt.

Zur Prüfung und zum Vergleich der Verbesserung der Gedächtnisleistung werden bei den einzelnen Gruppen der Versuchstiere 30 bzw. 60 Minuten vor dem Lerndurchgang oder innerhalb von 10 Sekunden nach Verabreichung des Fussschocks die Testsubstanzen in Dosen von 0,3 mg/kg, 3 mg/kg und 30 mg/kg (für jede Dosis werden 50 Mäuse verwendet) jeweils als Suspension in 0,5 % von Methylcellulose (Methocel) oder die Lösung alleine (= Placebo) intraperitoneal verabreicht. Die Verabreichung erfolgt entweder 30 (i.p.) bzw. 60 (p.o.) Minuten vor oder unmittelbar nach dem Versuch. 24 Stunden später wird anhand der Verweildauer jedes einzelnen Tieres auf der Plattform das Ausmass der Verbesserung oder Verschlechterung des Lerneffekts im Vergleich zu den Kontrolltieren, denen lediglich das Lösungsmittel verabreicht worden war, ermittelt. So lässt sich eine Verbesserung der Gedächtnisleistung z.B. durch i.p. Verabreichung von 2-(2-Isopropyl-aminopropyl)-5-pyridinol-monofumarat vor oder nach dem Lerndurchgang wie folgt nachweisen:

a) vor dem Versuch

| Dosis | Testlatenzen (sec) |
|---|---|
| 0,3 mg/kg i.p. | $35,3 \pm 5,8^{xx}$ |
| 3,0 mg/kg i.p. | $38,0 \pm 5,9^{xx}$ |
| 30,0 mg/kg i.p. | $56,5 \pm 7,9^{xxx}$ |
| Kontrollgruppe | $26,6 \pm 5,5$ |

b) unmittelbar nach dem Versuch

| Dosis | Testlatenzen (sec) |
|---|---|
| 0,3 mg/kg i.p. | $51,8 \pm 7,6^{xx}$ |
| 3,0 mg/kg i.p. | $46,4 \pm 7,2^{xx}$ |
| 30,0 mg/kg i.p. | $44,2 \pm 6,8^{xx}$ |
| Kontrollgruppe | $28,5 \pm 5,2$ |

$^{xx}$ $p < 0,01$

$^{xxx}$ $p < 0,001$ (Mann-Whitney U-Test)

Die Resultate zeigen, dass die Lernleistung schon mit einer Dosis von 0,3 mg/kg i.p. der oben genannten Verbindung bei Verabreichung unmittelbar nach dem Versuch um 78 %, und bei Verabreichung vor dem Versuch um 32 % verbessert wird. Diese Wirkungen sind statistisch hoch signifikant.

Beispiel 3: Herstellung von 10'000 Tabletten mit einem Gehalt von je 10 mg der aktiven Substanz:

| Bestandteile: | |
|---|---|
| 2-(2-Isopropylaminopropyl)-5-pyridinoldihydrochlorid | 100 g |
| Milchzucker | 3435 g |
| Maisstärke | 125 g |
| Polyäthylenglykol 6000 | 150 g |
| Talkpulver | 150 g |
| Magnesiumstearat | 40 g |
| Gereinigtes Wasser | q.s. |

Verfahren: Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 65 ml Wasser suspendiert und die Suspension zur siedenden Lösung von Polyäthylenglykol in 260 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 10,3 mm Durchmesser, welche eine Bruchrille aufweisen, gepresst.

Beispiel 4: Herstellung von 1000 Kapseln mit einem Gehalt von je 50 mg der aktiven Substanz.

| Bestandteile: | |
|---|---|
| 2-(2-Isopropylaminopropyl)-5-pyridinolmonofumarat | 50 g |
| Maisstärke | 5 g |
| Milchzucker | 143,75 g |
| Magnesiumstearat | 1 g |
| Netzmittel | 0,25 g |

Verfahren: Sämtliche pulverigen Bestandteile werden mit einem Sieb von 0,6 mm Maschenweite gesiebt. Dann wird der Wirkstoff zuerst mit Magnesiumstearat und Netzmittel und darauffolgend mit Stärke und Milchzucker in einem geeigneten Mischer homogenisiert. Kapseln Nr. 2 werden mit je 200 mg des Gemisches mit einer Füllmaschine gefüllt.

In analoger Weise werden Tabletten oder Kapseln, welche eine andere Verbindung Verbindung der Formel I enthalten, hergestellt.

**Patentansprüche**

**1.** Verwendung von sekundären 2-Aminoalkyl-5-pyridinolen der allgemeinen Formel I

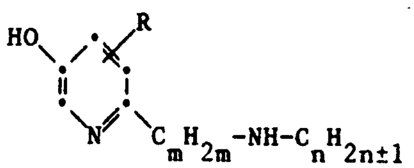

(I),

worin R Wasserstoff oder Alkyl mit höchstens 7 Kohlenstoffatomen bedeutet, m für eine ganze Zahl von 2 bis 4 steht, und n eine ganze Zahl von 1 bis 7 bedeutet, und ihren Säureadditionssalzen zur Herstellung eines nootropisch und antidepressiv wirksamen Arzneimittels.

**2.** Verwendung von Verbindungen der Formel I gemäss Anspruch 1, worin R Wasserstoff oder Methyl bedeutet, und die anderen Symbole die im Anspruch 1 angegebenen Bedeutungen haben, und ihren Säureadditionssalzen zur Herstellung eines nootropisch und antidepressiv wirksamen Arzneimittels.

**3.** Verwendung von Verbindungen der Formel I gemäss Anspruch 1, worin R Wasserstoff oder Methyl bedeutet, m für die ganze Zahl 2 oder 3 steht, und n eine ganze Zahl von 2 bis 6 bedeutet, und ihren Säureadditionssalzen zur Herstellung eines nootropisch und antidepressiv wirksamen Arzneimittels.

**4.** Verwendung von Verbindungen der allgemeinen Formel II

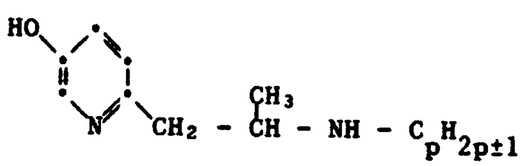

(II),

worin p eine ganze Zahl von 3 bis 6 bedeutet, und ihren Säureadditionssalzen zur Herstellung eines nootropisch und antidepressiv wirksamen Arzneimittels.

**5.** Verwendung von Verbindungen der Formel II gemäss Anspruch 4, worin $C_pH_{2p\pm1}$ Isopropyl oder tert.-Butyl bedeutet, und ihren Säureadditionssalzen zur Herstellung eines nootropisch und antidepressiv wirksamen Arzneimittels.

**6.** Verwendung von Verbindungen der Formel II gemäss Anspruch 4, worin $C_pH_{2p\pm1}$ Isopropyl, tert.-Butyl, Allyl oder Cyclopropyl bedeutet, und ihren Säureadditionssalzen zur Herstellung eines nootropisch und antidepressiv wirksamen Arzneimittels.

7. Verwendung von 2-(2-Isopropylaminopropyl)-5-pyridinol und seinen Säureadditionssalzen zur Herstellung eines nootropisch und antidepressiv wirksamen Arzneimittels.

8. Verwendung von Verbindungen der Formel I gemäss Anspruch 1, worin R, m und n die im Anspruch 1 genannten Bedeutungen haben, und ihren Säureadditionssalzen zur Herstellung eines Mittels zur Behandlung von Insuffizienz der cerebralen Leistung und Vigilanzregulation.

9. Verwendung von Verbindungen der Formel I gemäss Anspruch 1, worin R Wasserstoff oder Methyl bedeutet, m für die ganze Zahl 2 oder 3 steht und n eine ganze Zahl von 2 bis 6 bedeutet und ihren Säureadditionssalzen zur Herstellung eines Mittels zur Behandlung von Insuffizienz der cerebralen Leistung und Vigilanzregulation.

10. Verwendung von Verbindungen der Formel II gemäss Anspruch 4, worin p eine ganze Zahl von 3 bis 6 bedeutet und ihren Säureadditionssalzen zur Herstellung eines Mittels zur Behandlung von Insuffizienz der cerebralen Leistung und Vigilanzregulation.

11. Verwendung von Verbindungen der Formel II gemäss Anspruch 4, worin $C_pH_{2p\pm1}$ Isopropyl, tert.-Butyl, Allyl oder Cyclopropyl bedeutet, und ihren Säureadditionssalzen zur Herstellung eines Mittels zur Behandlung von Insuffizienz der cerebralen Leistung und Vigilanzregulation.

12. Verwendung von 2-(2-Isopropylaminopropyl)-5-pyridinol und seinen Säureadditionssalzen zur Herstellung eines Mittels zur Bekämpfung von Insuffizienz der cerebralen Leistung und Vigilanzregulation.

13. Verfahren zur Herstellung eines Mittels zur Behandlung von Insuffizienz der cerebralen Leistung und Vigilanzregulation, dadurch gekennzeichnet, dass man eine Verbindung der Formel I gemäss Anspruch 1, worin R, m und n die im Anspruch 1 angegebenen Bedeutungen haben, und ihre Säureadditionssalze, in eine zur Behandlung von im Anspruch 8 genannten Insuffizienzen geeignete Form bringt.

14. Verfahren zur Herstellung eines Mittels zur Behandlung von Insuffizienzen der cerebralen Leistung und Vigilanzregulation, dadurch gekennzeichnet, dass man eine Verbindung der Formel I gemäss Anspruch 1, worin R Wasserstoff oder Methyl bedeutet, m für die ganze Zahl 2 oder 3 steht und n eine ganze Zahl von 2 bis 6 bedeutet und ihre Säureadditionssalze, in eine zur Behandlung von im Anspruch 8 genannten Insuffizienzen geeignete Form bringt.

15. Verfahren zur Herstellung eines Mittels zur Behandlung von Insuffizienz der cerebralen Leistung und Vigilanzregulation, dadurch gekennzeichnet, das man eine Verbindung der Formel II gemäss Anspruch 4, worin p eine ganze Zahl von 3 bis 6 bedeutet und ihre Säureadditionssalze in eine zur Behandlung von im Anspruch 10 genannten Insuffizienzen geeignete Form bringt.

16. Verfahren zur Herstellung eines Mittels zur Behandlung von Insuffizienz der cerebralen Leistung und Vigilanzregulation, dadurch gekennzeichnet, dass man eine Verbindung der im Anspruch 4 gezeigten Formel II, worin $C_pH_{2p\pm1}$ Isopropyl, tert.-Butyl, Allyl oder Cyclopropyl bedeutet und ihre Säureadditionssalze, in eine zur Behandlung von im Anspruch 11 genannten Insuffizienzen geeignete Form bringt.

17. Verfahren zur Herstellung eines Mittels zur Behandlung von Insuffizienz der cerebralen Leistung und Vigilanzregulation, dadurch gekennzeichnet, dass man das 2-(2-Isopropylaminopropyl)-5-pyridinol und seine Säureadditionssalze in eine zur Behandlung von im Anspruch 12 genannten Insuffizienzen geeignete Form bringt.

## Claims

1. Use of a secondary 2-aminoalkyl-5-pyridinol of the general formula I

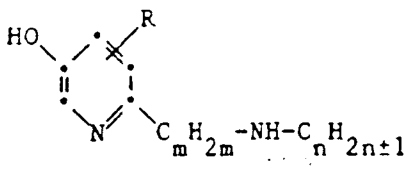 (I),

wherein R is hydrogen or alkyl having not more than 7 carbon atoms, m is an integer from 2 to 4 and n is an integer from 1 to 7, or an acid addition salt thereof, for the preparation of a medicament having nootropic and antidepressant activity.

2. Use of a compound of formula I according to claim 1, wherein R is hydrogen or methyl and the other symbols are as defined in claim 1, or an acid addition salt thereof, for the preparation of a medicament having nootropic and antidepressant activity.

3. Use of a compound of formula I according to claim 1, wherein R is hydrogen or methyl, m is the integer 2 or 3 and n is an integer from 2 to 6, or an acid addition salt thereof, for the preparation of a medicament having nootropic and antidepressant activity.

4. Use of a compound of the general formula II

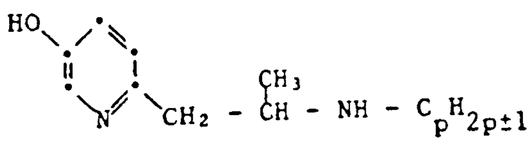 (II),

wherein p is an integer from 3 to 6, or an acid addition salt thereof, for the preparation of a medicament having nootropic and antidepressant activity.

5. Use of a compound of formula II according to claim 4, wherein $C_pH_{2p\pm1}$ is isopropyl or tert-butyl, or an acid addition salt thereof, for the preparation of a medicament having nootropic and antidepressant activity.

6. Use of a compound of formula II according to claim 4, wherein $C_pH_{2p\pm1}$ is isopropyl, tert-butyl, allyl or cyclopropyl, or an acid addition salt thereof, for the preparation of a medicament having nootropic and antidepressant activity.

7. Use of 2-(2-isopropylaminopropyl)-5-pyridinol, or an acid addition salt thereof, for the preparation of a medicament having nootropic and antidepressant activity.

8. Use of a compound of formula I according to claim 1, wherein R, m and n are as defined in claim 1, or an acid addition salt thereof, for the preparation of a composition for treating cerebral insufficiency and for regulating vigilance.

9. Use of a compound of formula I according to claim 1, wherein R is hydrogen or methyl, m is the integer 2 or 3 and n is an integer from 2 to 6, or an acid addition salt thereof, for the preparation of a composition for treating cerebral insufficiency and for regulating vigilance.

10. Use of a compound of formula II according to claim 4, wherein p is an integer from 3 to 6, or an acid addition salt thereof, for the preparation of a composition for treating cerebral insufficiency and for regulating vigilance.

11. Use of a compound of formula II according to claim 4, wherein $C_pH_{2p\pm1}$ is isopropyl, tert-butyl, allyl or cyclopropyl, or an acid addition salt thereof, for the preparation of a composition for treating cerebral insufficiency and for regulating vigilance.

**12.** Use of 2-(2-isopropylaminopropyl)-5-pyridinol, or an acid addition salt thereof, for the preparation of a composition for treating cerebral insufficiency and for regulating vigilance.

**13.** A process for the preparation of a composition for treating cerebral insufficiency and for regulating vigilance, which comprises bringing a compound of formula I according to claim 1 wherein R, m and n are as defined in claim 1, or an acid addition salt thereof, into a form suitable for the treatment of the insufficiencies mentioned in claim 8.

**14.** A process for the preparation of a composition for treating cerebral insufficiency and for regulating vigilance, which comprises bringing a compound of formula I according to claim 1 wherein R is hydrogen or methyl, m is the integer 2 or 3 and n is an integer from 2 to 6, or an acid addition salt thereof, into a form suitable for the treatment of the insufficiencies mentioned in claim 8.

**15.** A process for the preparation of a composition for treating cerebral insufficiency and for regulating vigilance, which comprises bringing a compound of formula II according to claim 4 wherein p is an integer from 3 to 6, or an acid addition salt thereof, into a form suitable for the treatment of the insufficiencies mentioned in claim 10.

**16.** A process for the preparation of a composition for treating cerebral insufficiency and for regulating vigilance, which comprises bringing a compound of formula II shown in claim 4, wherein $C_pH_{2p\pm1}$ is isopropyl, tert-butyl, allyl or cyclopropyl, or an acid addition salt thereof, into a form suitable for the treatment of the insufficiencies mentioned in claim 11.

**17.** A process for the preparation of a composition for treating cerebral insufficiency and for regulating vigilance, which comprises bringing 2-(2-isopropylaminopropyl)-5-pyridinol, or an acid addition salt thereof, into a form suitable for the treatment of the insufficiencies mentioned in claim 12.

**Revendications**

**1.** Application de 2-aminoalcoyl-5-pyridinols secondaires de formule générale I

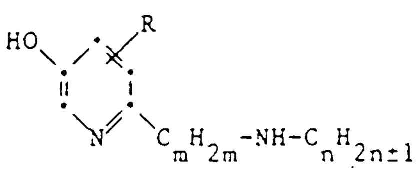

(I),

dans laquelle R représente un hydrogène ou un alcoyle ayant au plus 7 atomes de carbone, m représente un nombre entier allant de 2 à 4, et n représente un nombre entier allant de 1 à 7, et de leurs sels d'addition d'acides, à la préparation d'un médicament à action nootropique et antidépressante.

**2.** Application de composés de formule I selon la revendication 1, dans laquelle R représente un hydrogène ou un méthyle, et les autres symboles ont les significations données dans la revendication 1, et de leurs sels d'addition d'acides à la préparation d'un médicament à action nootropique et antidépressante.

**3.** Application de composés de formule I selon la revendication 1, dans laquelle R représente un hydrogène ou un méthyle, m représente le nombre entier 2 ou 3, et n représente un nombre entier allant de 2 à 6, et de leurs sels d'addition d'acides, à la préparation d'un médicament à action nootropique et antidépressante.

**4.** Application de composés de formule générale II

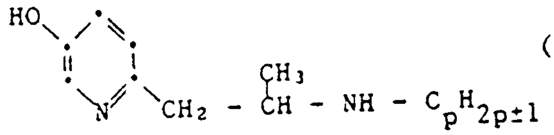

(II),

dans laquelle p représente un nombre entier valant de 3 à 6, et de leurs sels d'addition d'acides, à la préparation d'un médicament à action nootropique et antidépressante.

**5.** Application de composés de formule II selon la revendication 4, dans laquelle $C_pH_{2p\pm1}$ représente un isopropyle ou un tert.-butyle, et de leurs sels d'addition d'acides, à la préparation d'un médicament à action nootropique et antidépressante.

**6.** Application de composés de formule II selon la revendication 4, dans laquelle $C_pH_{2p\pm1}$ représente un isopropyle, tert.-butyle, allyle ou cyclopropyle, et de leurs sels d'addition d'acides, à la préparation d'un médicament à action nootropique et antidépressante.

**7.** Application de 2-(2-isopropylaminopropyl)-5-pyridinol et de ses sels d'addition d'acides à la préparation d'un médicament à action nootropique et antidépressante.

**8.** Application de composés de formule I selon la revendication 1, dans laquelle R, m et n ont les significations données dans la revendication 1, et de leurs sels d'addition d'acides, à la préparation d'un médicament pour le traitement de l'insuffisance de fonctionnalité cérébrale et de la régulation de la vigilance.

**9.** Application de composés de formule I selon la revendication 1, dans laquelle R représente un hydrogène ou un méthyle, m représente le nombre entier 2 ou 3, et n représente un nombre entier allant de 2 à 6, et de leurs sels d'addition d'acides, à la préparation d'un médicament pour le traitement de l'insuffisance de la fonctionnalité cérébrale et de la régulation de la vigilance.

**10.** Application de composés de formule II selon la revendication 4, dans laquelle p représente un nombre entier allant de 3 à 6, et de leurs sels d'addition d'acides, à la préparation d'un agent pour le traitement de l'insuffisance de la fonctionnalité cérébrale et de la régulation de la vigilance.

**11.** Application de composés de formule II selon la revendication 4, dans laquelle $C_pH_{2p\pm1}$ représente un isopropyle, tert.-butyle, allyle ou cyclopropyle et de leurs sels d'addition d'acides à la préparation d'un agent pour le traitement de l'insuffisance de la fonctionnalité cérébrale et de la régulation de la vigilance.

**12.** Application de 2-(2-isopropylaminopropyl)-5-pyridinol et de ses sels d'addition d'acides à la préparation d'un agent pour combattre l'insuffisance de la fonctionnalité cérébrale et de la régulation de la vigilance.

**13.** Procédé de préparation d'un agent pour le traitement de l'insuffisance de la fonctionnalité cérébrale et de la régulation de la vigilance, caractérisé en ce qu'on met sous une forme appropriée au traitement des insuffisances mentionnées dans la revendication 8, un composé de formule I selon la revendication 1, dans lequel R, m et n ont les significations données dans la revendication 1, et ses sels d'addition d'acides.

**14.** Procédé de préparation d'un agent pour le traitement de l'insuffisance de la fonctionnalité cérébrale et de la régulation de la vigilance, caractérisé en ce qu'on met sous une forme appropriée au traitement des insuffisances mentionnées dans la revendication 8, un composé de formule I selon la revendication 1, dans lequel R représente un hydrogène ou un méthyle, m représente le nombre entier 2 ou 3, et n représente un nombre entier allant de 2 à 6, et ses sels d'addition d'acides.

**15.** Procédé de préparation d'un agent de traitement de l'insuffisance de la fonctionnalité cérébrale et de la régulation de la vigilance, caractérisé en ce qu'on met sous une forme appropriée à l'insuffisance mentionnée dans la revendication 10 un composé de formule II selon la revendication 4, dans laquelle p représente un nombre entier valant de 3 à 6 et ses sels d'addition d'acides.

**16.** Procédé de préparation d'un agent de traitement de l'insuffisance de la fonctionnalité cérébrale et de la régulation de la vigilance, caractérisé en ce qu'on met sous une forme appropriée au traitement des insuffisances mentionnées dans la revendication 11 un composé de formule II présenté dans la revendication 4, dans lequel $C_pH_{2p\pm1}$ représente un isopropyle, tert.-butyle, allyle ou cyclopropyle, et ses sels d'addition d'acides.

**17.** Procédé de préparation d'un agent pour le traitement de l'insuffisance de la fonctionnalité cérébrale et de la régulation de la vigilance, caractérisé en ce qu'on met sous une forme appropriée au traitement des insuffisances mentionnées dans la revendication 12, le 2-(2-isopropylaminopropyl)-5-pyridinol et ses d'addition d'acides.